(19) Europäisches Patentamt
European Patent Office
Office européen des brevets

(11) Veröffentlichungsnummer: **0 172 502**
**B1**

(12) **EUROPÄISCHE PATENTSCHRIFT**

(45) Veröffentlichungstag der Patentschrifft: **17.01.90**

(51) Int. Cl.⁴: **C 07 D 303/36**, C 07 D 331/02, C 07 D 493/04

(21) Anmeldenummer: **85109992.9**

(22) Anmeldetag: **08.08.85**

(54) **Cyclohexenonderivate, Verfahren zu ihrer Herstellung und ihre Verwendung zur Bekämpfung unerwünschten Pflanzenwuchses.**

(30) Priorität: **18.08.84 DE 3430483**
**25.01.85 DE 3502391**

(43) Veröffentlichungstag der Anmeldung:
**26.02.86 Patentblatt 86/09**

(45) Bekanntmachung des Hinweises auf die Patenterteilung:
**17.01.90 Patentblatt 90/03**

(84) Bennante Vertragsstaaten:
**BE CH DE FR GB IT LI NL**

(56) Entgegenhaltungen:
**DE-A-2 439 104**
**DE-A-3 032 973**
**DE-B-2 356 892**

(73) Patentinhaber: **BASF Aktiengesellschaft**
**Carl-Bosch-Strasse 38**
**D-6700 Ludwigshafen (DE)**

(72) Erfinder: **Jahn, Dieter, Dr.**
**Burgunder Weg 8**
**D-6803 Edingen-Neckarhausen (DE)**
Erfinder: **Becker, Rainer, Dr.**
**Im Haseneck 22**
**D-6702 Bad Duerkheim (DE)**
Erfinder: **Keil, Michael, Dr.**
**Fontanestrasse 4**
**D-6713 Freinsheim (DE)**
Erfinder: **Schirmer, Ulrich, Dr.**
**Berghalde 79**
**D-6900 Heidelberg (DE)**
Erfinder: **Wuerzer, Bruno, Dr.**
**Ruedigerstrasse 13**
**D-6701 Otterstadt (DE)**
Erfinder: **Meyer, Norbert, Dr.**
**Dossenheimer Weg 22**
**D-6802 Ladenburg (DE)**

**EP 0 172 502 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft Cyclohexenonderivate, Verfahren zu ihrer Herstellung, Herbizide, die diese Verbindungen als Wirkstoff enthalten, sowie ein Verfahren zur Bekämpfung unerwünschten Pflanzenwuchses.

Es ist bekannt, Cyclohexenonderivate zur Bekämpfung von unerwünschten Gräsern in breitblättrigen Kulturen zu verwenden (DE-A-2 439 104). Darüber hinaus sind aus DE-A-3 032 973 in 5-Stellung cycloalkenylsubstituierte Derivate bekannt, die ebenfalls herbizid wirksam sind.

Es wurde gefunden, daß Cyclohexenonderivate der Formel I

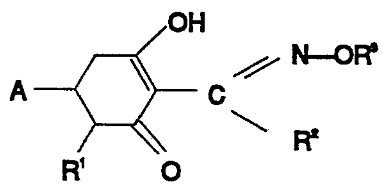

(I),

in der

A ein Cycloalkylrest mit 5 bis 12 Ringgliedern, an den ein oder zwei Oxiran- oder Thiiranringe kondensiert sind, und der gegebenenfalls durch bis zu vier Methylgruppen substituiert und/oder durch eine Alkylenkette mit maximal 3 C-Atomen überbrückt ist,
$R_1$ Wasserstoff, Methoxycarbonyl oder Cyano, wobei Wasserstoff bevorzugt ist,
$R_2$ Alkyl mit 1 bis 4 Kohlenstoffatomen und
$R_3$ Alkyl mit 1 bis 3 C-Atomen, Alkenyl mit 3 oder 4 C-Atomen, Halogenalkenyl mit 3 oder 4 C-Atomen und 1 bis 3 Halogensubstituenten oder Propargyl bedeuten,

und Salze dieser Verbindungen eine gute herbizide Wirkung, vorzugsweise gegen Arten aus der Familie der Gräser (Gramineen), haben. Sie sind selektiv in breitblättrigen Kulturen sowie in monokotylen Gewächsen, welche nicht zu den Gramineen zählen.

Die Verbindungen der Formel I können in mehreren Formen auftreten, die alle vom Patentanspruch umfaßt werden:

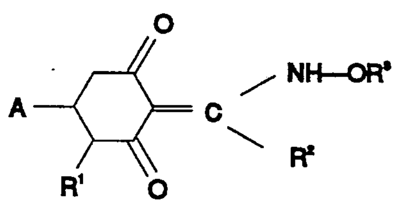

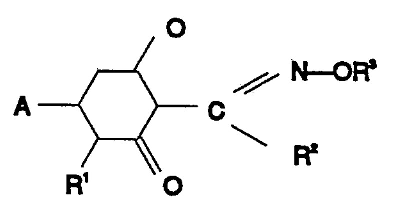

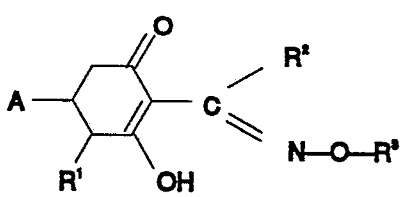

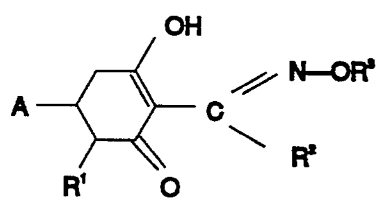

A in Formel I steht für einen Cycloalkylrest mit 5 bis 12 Ringgliedern, vorzugsweise 5 bis 8 Ringgliedern, an den ein oder zwei Oxiran- oder Thiiranringe kondensiert sind, und der gegebenenfalls durch bis zu vier Methylgruppen substituiert und durch eine Alkylenkette mit maximal 3 C-Atomen überbrückt ist, beispielsweise Epoxycyclopentyl, Epoxycyclohexyl, Epoxycycloheptyl, Epoxycyclooctyl, Epoxycyclododecyl, Diepoxycyclododecyl, Epoxymethylcyclopentyl, Dimethylepoxycyclopentyl, Epoxymethylcyclohexyl, Dimethylepoxycyclohexyl, Epoxytrimethylcyclohexyl, Epoxytetramethylcyclohexyl, Epoxybicycloheptyl, Diepoxytrimethylcyclohexyl, Epoxydimethylbicycloheptyl, Epithiocyclopentyl, Epithiocyclohexyl, Epithiobicycloheptyl. Der 3,4-Epoxycyclopentylrest ist besonders bevorzugt.

$R_2$ in Formel I steht für unverzweigte oder verzweigte Alkylreste mit 1 bis 4, vorzugsweise 2 oder 3 Kohlenstoffatomen, d.h. für Methyl, Ethyl, n-Propyl, i-Propyl, n-Butyl, sec-Butyl, i-Butyl, tert-Butyl.

Reste für $R_3$ in Formel I sind Propargyl, Alkyl mit 1 bis 3 C-Atomen, Alkenyl mit 3 oder 4 C-Atomen oder Halogenalkenyl mit 3 oder 4 C-Atomen, das bis zu drei Halogensubstituenten enthalten kann, beispielsweise Methyl, Ethyl, n-Propyl, i-Propyl, n-Butyl, sec-Butyl, i-Butyl, tert-Butyl, Allyl, 3-Chlor-prop-2-enyl, 2-Chlor-prop-2-enyl, 1,3-Dichlor-prop-2-enyl, 2,3,3-Trichlor-prop-2-enyl.

Als Salze der Verbindung der Formel I kommen landwirtschaftlich brauchbare Salze, beispielsweise die Alkalimetallsalze, insbesondere die Kalium- oder Natriumsalze, Erdalkalimetallsalze, insbesondere Calcium-, Magnesium- oder Bariumsalze, Mangan-m, Kupfer-, Zink- oder Eisensalze sowie Ammonium-, Sulfonium- und Phosphoniumsalze in Betracht.

Die Verbindungen der Formel I können durch Umsetzung von Tricarbonylverbindungen der Formel II

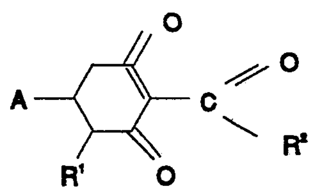

(II),

in der A, $R^1$ und $R^2$ die obengenannten Bedeutungen haben, mit Hydroxylaminderivaten $R^3O\text{-}NH_3Y$, in der $R^3$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80°C oder zwischen 0°C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Als Verdünnungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, cyclische Ether, wie Dioxan, Tetrahydrofuran, geeignet. Auch Gemische dieser Verdünnungsmittel können verwendet werden.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R^3O\text{-}NH_2$, in der $R^3$ die obengenannten Bedeutungen hat, in inerten Verdünnungsmitteln bei einer Temperatur zwischen 0°C und dem Siedepunkt des Reaktionsgemisches, insbesondere zwischen 15 und 70°C, erhalten werden. Gegebenenfalls kann das Hydroxylamin in wäßriger Lösung eingesetzt werden.

Geeignete Verdünnungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, beispielsweise die Mangan-, Kupfer-, Zink-, Eisen-, Calcium-, Magnesium- und Bariumsalze können aus den Natriumsalzen durch Reaktion mit den entsprechenden Metallchloriden in wäßriger Lösung hergestellt werden. Ammonium-, Sulfonium- und Phosphoniumsalze können durch Umsetzung von Verbindungen der Formel I mit Ammonium-, Sulfonium- oder Phosphoniumhydroxiden gegebenenfalls in wäßriger Lösung hergestellt werden.

Die Tricarbonylverbindungen der Formel II sind neu. Sie können aus Cyclohexan-1,3-dionen der Formel III, die auch in den tautomeren Formen IIIa und IIIb vorliegen können,

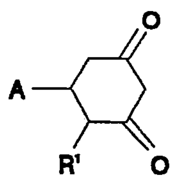

(III)

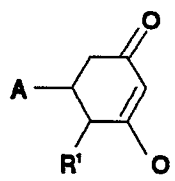

(III a)

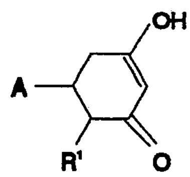

(III b)

nach literaturbekannten Methoden (Tetrahedron Letters, *29*, 2491 (1975)) hergestellt werden.

Es ist auch möglich, die neuen Verbindungen der Formel II über die Zwischenstufe der Enolester, die bei der Umsetzung von Verbindungen der Formel III eventuell als Isomerengemisch anfallen und in Gegenwart von Imidazol- oder Pyridinderivaten umgelagert werden (JP-A-5 515 890), herzustellen.

Wie aus dem Vorangehenden ersichtlich, stellen die Tricarbonylverbindungen der Formel II wertvolle Zwischenprodukte für die Herstellung der herbizid wirksamen Cyclohexenonderivate der Formel I dar.

Zu den Verbindungen der Formel III gelangt man nach literaturbekannten Verfahren, wie dies aus folgendem Schema hervorgeht:

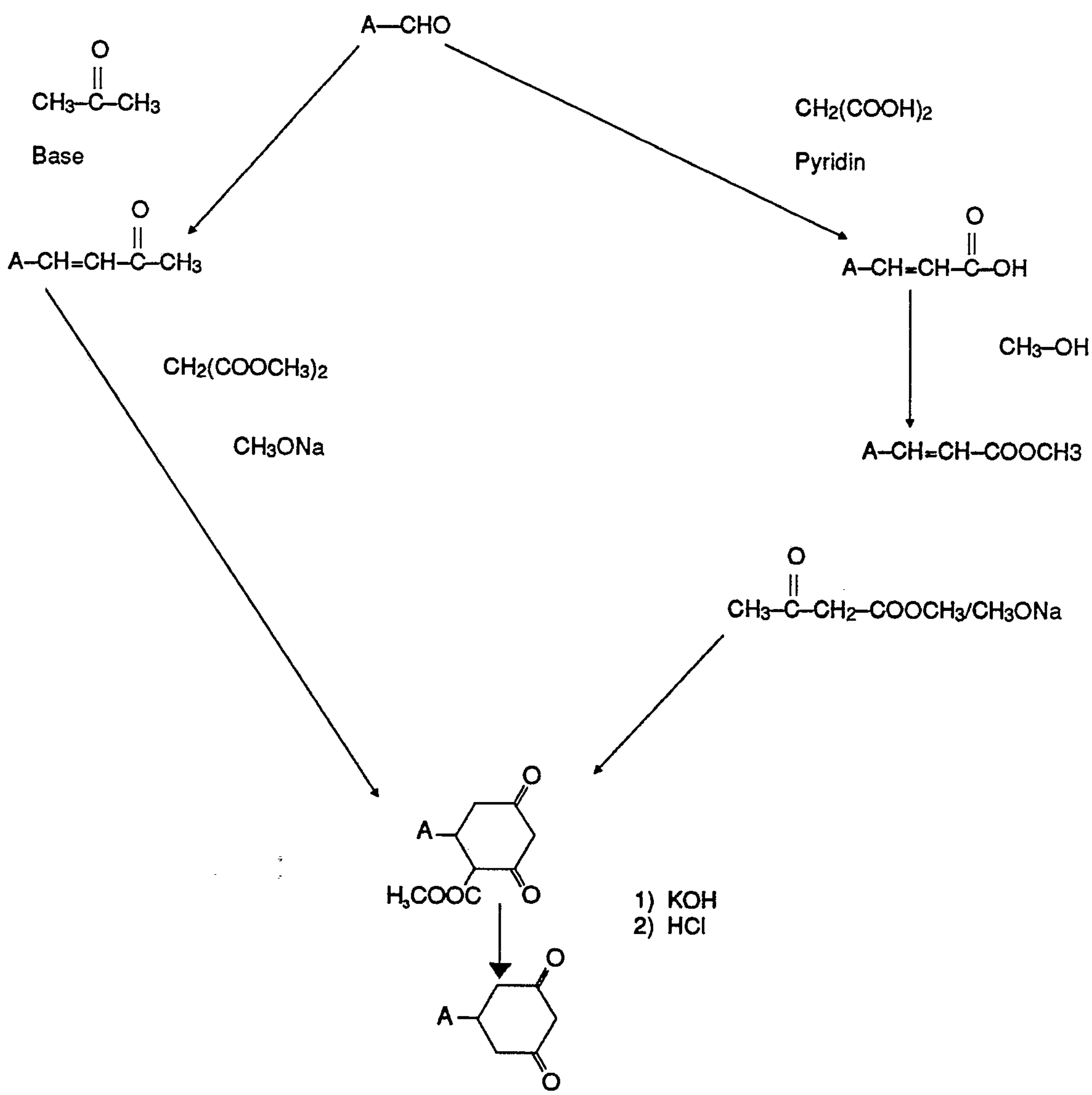

Aldehyde der allgemeinen Formel A-CHO können aus den entsprechenden ungesättigten Aldehyden nach literaturbekannten Verfahren erhalten werden, wobei es notwendig sein kann, die Aldehyd-Funktion zu schützen.

Die Oxiran-Derivate kann man in an sich bekannter Weise durch Umsetzung entsprechender ungesättigter Aldehyde oder Aldehyd-Derivate mit Peroxyverbindungen, wie Wasserstoffperoxid, tert-Butylhydroperoxid, Perameisensäure, m-Chlorperbenzoesäure, oder Luftsauerstoff erhalten.

Die Epoxid-Struktur läßt sich auch durch Halogenwasserstoffabspaltung aus 1,2-Halogenhydrinen synthetisieren.

Die Thiiran-Verbindungen können entweder aus den entsprechenden Epoxiden mit Thiocyanaten oder Thioharnstoff, wie dies z. B. in J. Chem. Soc. *1946*, 1050 beschrieben ist, oder aus den entsprechenden ungesättigten Derivaten durch Umsetzung mit Schwefel-Transferreagentien, wie Arylthiosulfenylchloriden (Chemistry of Heterocyclic Compounds Bd. 42, Seite 340). Die vorstehend aufgeführten Umwandlungsmethoden können gegebenenfalls auf jeder Synthesestufe vorgenommen werden.

Die folgenden Beispiele erläutern die Herstellung der Cyclohexenonderivate der Formel I. In den Beispielen verhalten sich Gewichtsteile zu Volumenteilen wie Kilogramm zu Liter.

Die $^1$H-NMR-Spektren werden in Deuterochloroform als Lösungsmittel mit Tetramethylsilan als innerem Standard aufgenommen. Die $^1$H-chemischen Verschiebungen sind jeweils in [ppm] angegeben. Für die Signalstruktur werden folgende Abkürzungen benützt:

s = Singulett, d = Dublett, t = Triplett, q = Quartett, m = Multiplett, stärkstes Signal

**Beispiel 1**

3,5 Gewichtsteile 2-Butyryl-5-(3,4-epoxycyclohexyl)-cyclohexan-1,3-dion, 1,5 Gewichtsteile Allyloxyammoniumchlorid und 1,2 Gewichtsteile Natriumhydrogencarbonat wurden in 50 Volumenteilen Methanol 16 Stunden bei Raumtemperatur gerührt. Das Lösungsmittel wurde unter vermindertem Druck abdestilliert, der Rückstand mit je 50 Volumenteilen Wasser und Dichlormethan gerührt, die organische Phase abgetrennt, die wäßrige Phase einmal mit 50 Volumenteilen Dichlormethan extrahiert, die vereinigten organischen Phasen über Natriumsulfat getrocknet und das Lösungsmittel unter vermindertem Druck abdestilliert. Man erhielt 2-(1-Allyloxyimino-butyl)-5-(3,4-epoxycyclohexyl)-3-hydroxy-cyclohex-2-en-1-on (Wirkstoff Nr 1).

$^1$H-NMR-Spektrum:

= 0,95 (t), 2,9 (q), 5,3 (m)

**Beispiel 2**

3,5 Gewichtsteile 2-Butyryl-5-(3,4-epoxycyclohexyl)-cyclohexan-1,3-dion und 0,8 Gewichtsteile Ethoxyamin wurden in 100 Volumenteilen Methanol bei Raumtemperatur 16 Stunden gerührt. Das Lösungsmittel wurde unter vermindertem Druck abdestilliert, der Rückstand in Dichlormethan gelöst, die Lösung mit 5 gew.-%-iger Salzsäure und Wasser gewaschen, über Natriumsulfat getrocknet und das Lösungsmittel unter vermindertem Druck abdestilliert. Man erhielt 2-(1-Ethoxyimino-butyl)-5-(3,4-epoxycyclohexyl)-3-hydroxycyclohex-2-en-1-on (Wirkstoff Nr. 2).

$^1$H-NMR-Spektrum:

= 1,3 (t), 3,2 (m), 4,1 (d)

**Beispiel 3**

9 Gewichtsteile 2-(1-Propoxyimino-butyl)-5-(cyclohex-3-enyl)-3-hydroxycyclohex-2-en-1-on wurden in 100 Gewichtsteilen Dichlormethan gelöst und bei 5 - 10°C 5,8 Gewichtsteile m-Chlorperbenzoesäure (ca. 80 %) in 100 Volumenteilen Dichlormethan zugetropft. Nach einer Stunde wurde mittels Dünnschichtchromatographie der Reaktionsverlauf überprüft (DC-Fertigplatten Kieselgel 60, Laufmittel Cyclohexan/Essigsäureethylester 1 : 1). (Falls noch Ausgangsprodukt vorhanden ist, wird noch weitere m-Chlorperbenzoesäure bis zur vollständigen Umsetzung zugetropft.) Überschüssige Persäure wurde durch Schütteln mit Natriumthiosulfatlösung vernichtet. Daraufhin wurde zweimal mit halbgesättigter Natriumhydrogencarbonat-Lösung und einmal mit Wasser extrahiert, die organische Phase über Natriumsulfat getrocknet und das Lösungsmittel unter vermindertem Druck abdestilliert. Man erhielt 2-(1-Propoxyimino-2-butyl)-5-(3,4-epoxycyclohexyl)-3-hydroxycyclohex-2-en-1-on (Wirkstoff Nr. 3).

= 2,2 (m), 3,1 (m), 4,0 (q)

Die folgenden Verbindungen erhält man wie in den Beispielen erläutert.

| Wirkstoff Nr. | A | $R^1$ | $R^2$ | $R^3$ | $^1$H-NMR-Daten |
|---|---|---|---|---|---|
| 4 | 3,4-Epoxy-1-methyl-cyclohexyl | H | n-Propyl | Ethyl | 0,85 (s),1,3 (t),2,9 (m) |
| 5 | 3,4-Epoxy-1-methyl-cyclohexyl | H | n-Propyl | Allyl | |
| 6 | 3,4-Epoxy-4-methyl-cyclohexyl | H | n-Propyl | Ethyl | 1,0 (t),1,3 (s),2,5 (m) |
| 7 | 3,4-Epoxy-4-methyl-cyclohexyl | H | n-Propyl | Allyl | |
| 8 | 3,4-Epoxy-6-methyl-cyclohexyl | H | n-Propyl | Ethyl | 0,95 (t),1,6 (m),4,1 (q) |
| 9 | 3,4-Epoxy-6-methyl-cyclohexyl | H | n-Propyl | Allyl | 0,9 (m),2,9 (d),4,55 (d) |
| 10 | 3,4-Epoxy-6-methyl-cyclohexyl | H | n-Propyl | 3-Chlorallyl (trans) | |
| 11 | 1,2-Epoxy-2,6,6-trimethyl-cyclohexyl | H | n-Propyl | Ethyl | 1,40 (s),2,9 (m),4,1 (q) |
| 12 | 1,2-Epoxy-2,6,6-trimethyl-cyclohexyl | H | n-Propyl | Ethyl | |
| 13 | 3,4-Dimethyl-3,4-epoxy-cyclohexyl | H | n-Propyl | Ethyl | 0,95 (t),1,35 (m),4,10 (q) |
| 14 | 3,4-Dimethyl-3,4-epoxy-cyclohexyl | H | n-Propyl | Allyl | |
| 15 | 1,2-Epoxy-cyclooctyl | H | n-Propyl | Ethyl | 1,0 (t),1,30 (t),4,1 (q) |
| 16 | 1,2-Epoxy-cyclooctyl | H | n-Propyl | Allyl | 0,95 (t),1,35 (s),2,95 (m) |
| 17 | 4,5-8,9-Diepoxy-cyclododecyl | H | n-Propyl | Ethyl | |
| 18 | 4,5-8,9-Diepoxy-cyclododecyl | H | n-Propyl | Allyl | |
| 19 | 3,4-Epoxy-cyclopentyl | H | n-Propyl | Ethyl | |
| 20 | 3,4-Epoxy-cyclopentyl | H | n-Propyl | Allyl | |
| 21 | 3,4-Epoxy-cyclopentyl | H | n-Propyl | 3-Chlorallyl (trans) | |
| 22 | 3,4-Epoxy-cyclopentyl | H | n-Propyl | Propargyl | |
| 23 | 3,4-Epoxy-cyclopentyl | H | Ethyl | Ethyl | |
| 24 | 5,6-Epoxy-bicyclo [2.2.1]-hept-2-yl | H | Ethyl | Propargyl | |
| 25 | 5,6-Epoxy-bicyclo [2.2.1]-hept-2-yl | H | Ethyl | Allyl | 2,5 (m),2,9 (m),4,55 (d) |
| 26 | 5,6-Epoxy-bicyclo [2.2.1]-hept-2-yl | H | Ethyl | Ethyl | 0,8 (d)2,5 (m),2,9 (m) |
| 27 | 5,6-Epoxy-bicyclo [2.2.1]-hept-2-yl | H | n-Propyl | 3-Chlorallyl (trans) | 3,15 (d),4,5 (d),6,3 (m) |
| 28 | 5,6-Epoxy-bicyclo [2.2.1]-hept-2-yl | H | n-Propyl | Ethyl | 1,3 (t),2,9 (s),4,1 (q) |
| 29 | 5,6-Epoxy-bicyclo [2.2.1]-hept-2-yl | H | n-Propyl | Allyl | 1,0 (t),2,5 (m),4,5 (d) |
| 30 | 5,6-Epoxy-bicyclo [2.2.1]-hept-2-yl | H | n-Propyl | Propargyl | 1,0 (t),2,6 (m),4,65 (s) |
| 31 | 2,3-Epoxy-cyclohexyl | H | n-Propyl | Ethyl | |
| 32 | 2,3-Epoxy-cyclohexyl | H | n-Propyl | Allyl | |
| 33 | 3,4-Epithio-cyclohexyl | H | n-Propyl | Ethyl | 0,95 (t),2,90 (d),3,15 (m) |
| 34 | 3,4-Epithio-cyclohexyl | H | n-Propyl | Allyl | |
| 35 | 5,6-Epithio-bicyclo-[2.2.1]hept-2-yl | H | n-Propyl | Ethyl | |
| 36 | 5,6-Epithio-bicyclo-[2.2.1]hept-2-yl | H | n-Propyl | Allyl | |
| 37 | 3,4-Epithio-cyclopentyl | H | n-Propyl | Ethyl | |
| 38 | 3,4-Epithio-cyclopentyl | H | n-Propyl | Allyl | |
| 39 | 3,4-Epithio-cyclopentyl | H | Ethyl | Allyl | |
| 40 | 3,4-Epithio-cyclopentyl | H | Ethyl | Ethyl | |
| 41 | 2,3-Epithio-cyclopentyl | H | Ethyl | Ethyl | |
| 42 | 2,3-Epithio-cyclopentyl | H | Ethyl | Allyl | |
| 43 | 2,3-Epithio-cyclopentyl | H | n-Propyl | Allyl | |
| 44 | 2,3-Epithio-cyclopentyl | H | n-Propyl | Ethyl | |
| 45 | 2,3-Epithio-cyclopentyl | H | n-Propyl | 3-Chlorallyl (trans) | |
| 46 | 2,3-Epithio-cyclohexyl | H | n-Propyl | Ethyl | |
| 47 | 2,3-Epithio-cyclohexyl | H | n-Propyl | Allyl | |
| 48 | 2,3-Epithio-cyclohexyl | H | Ethyl | Ethyl | |
| 49 | 2,3-Epithio-cyclohexyl | H | Ethyl | Allyl | |
| 50 | 4,5-Epoxy-cyclooctyl | H | n-Propyl | Ethyl | 1,0 (t),1,35 (t),4,1 (q) |
| 51 | 4,5-Epoxy-cyclooctyl | H | n-Propyl | Allyl | 1,0 (t),2,9 (m),4,55 (d) |
| 52 | 4,5-Epoxy-cyclooctyl | H | Ethyl | Ethyl | 1,35 (t),2,9 (m),4,1 (q) |
| 53 | 4,5-Epoxy-cyclooctyl | H | Ethyl | Allyl | 1,15 (t),2,9 (m),4,5 (d) |
| 54 | 4,5-Epoxy-cyclooctyl | H | Ethyl | 3-Chlorallyl (trans) | 1,15 (t),2,9 (m),4,55 (d) |
| 55 | 4,5-Epoxy-cyclooctyl | H | n-Propyl | 3-Chlorallyl (trans) | 0,95 (t),1,5 (m),4,55 (d) |
| 56 | 4,5-8,9-Diepoxy-cyclododecyl | H | Ethyl | Ethyl | 1,2 (t),1,3 (m),4,1 (q) |
| 57 | 3,4-Epoxy-6-methyl-cyclohexyl | H | Ethyl | Ethyl | 1,35 (t),2,9 (q),4,1 (q) |
| 58 | 3,4-Epoxy-6-methyl-cyclohexyl | H | Ethyl | Allyl | 1,15 (t),2,9 (m),4,5 (d) |
| 59 | 2,6-Dimethyl-3,4-epoxycyclohexyl | H | n-Propyl | Ethyl | 1,3 (t),2,9 (s),4,1 (q) |

| Wirkstoff Nr. | A | $R^1$ | $R^2$ | $R^3$ | $^1$H-NMR-Daten |
|---|---|---|---|---|---|
| 60 | 2,6-Dimethyl-3,4-epoxycyclohexyl | H | n-Propyl | 3-Chlorallyl (trans) | 1,15 (t),2,8 (m),6,35 (m) |
| 61 | 5,6-Epoxy-bicyclo [2.2.1]-hept-2-yl | H | Ethyl | 3-Chlorallyl (trans) | 1,15 (t),2,5 (m),4,55 (d) |
| 62 | 3,4-Epoxy-cyclohexyl | H | Ethyl | Ethyl | 1,3 (t),3,15 (m),4,1 (q) |
| 63 | 3,4-Epoxy-cyclohexyl | H | Ethyl | Allyl | 1,15 (t),2,85 (d),4,55 (d) |
| 64 | 3,4-Epoxy-cyclohexyl | H | Ethyl | 3-Chlorallyl (trans) | 1,1 (t),2,85 (d),6,3 (d) |
| 65 | 3,4-Epoxy-2-methylcyclohexyl | H | n-Propyl | Ethyl | 1,6 (q),3,2 (s),4,1 (q) |
| 66 | 3,4-Epoxy-2-methylcyclohexyl | H | n-Propyl | Allyl | 0,95 (t),1,85 (m),3,20 (s) |
| 67 | 3,4-Epoxy-2-methylcyclohexyl | H | Ethyl | Ethyl | 1,3 (t),2,35 (m),4,1 (q) |
| 68 | 3,4-Epoxy-2-methylcyclohexyl | H | Ethyl | Allyl | 2,4 (m),4,6 (d),6,0 (m) |
| 69 | 3,4-Epoxy-2-methylcyclohexyl | H | Ethyl | 3-Chlorallyl (trans) | 1,15 (t),2,9 (q),4,55 (d) |
| 70 | 3,4-Epoxy-cyklopentyl | H | Ethyl | Allyl | |
| 71 | 3,4-Epoxy-cyklopentyl | H | Ethyl | 3-Chlorallyl (trans) | |
| 72 | 4,5-Epoxy-cycloheptyl | H | Ethyl | Ethyl | |
| 73 | 4,5-Epoxy-cykloheptyl | H | Ethyl | Allyl | |
| 74 | 4,5-Epoxy-cycloheptyl | H | Ethyl | 3-Chlorallyl (trans) | |
| 75 | 4,5-Epoxy-cycloheptyl | H | n-Propyl | 3-Chlorallyl (trans) | |
| 76 | 4,5-Epoxy-cycloheptyl | H | n-Propyl | Ethyl | |
| 77 | 4,5-Epoxy-cycloheptyl | H | n-Propyl | Allyl | |

Die Cyclohexenonderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, z. B. Ammoniumsulfat, Ammoniumphosphat, Ammoniumnitrat, Harnstoffe und pflanzliche Produkte, wie Getreidemehl, Baumrinden-, Holz-und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteile Calciumsalz der Dodecylbenzolsulfonsäure und 5 Gewichtsteile des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile des Wirkstoffs Nr. 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöls besteht. Durch Eingießen und feines Verteilen der Lösung in 100 000 Gewichtsteilen Wasser erhält man eine wäßrige Dispersion, die 0,02 Gew.-% des Wirkstoffs enthält.

V. 20 Gewichtsteile des Wirkstoffs Nr. 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile des Wirkstoffs Nr. 13 werden mit 97 Gewichtsteilen feinteiligem Kaolin vermischt. Man erhält auf diese Weise ein Stäubemittel, das 3 Gew.-% des Wirkstoffs enthält.

VII. 30 Gewichtsteile des Wirkstoffs Nr. 11 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teile eines paraffinischen Mineralöls innig vermischt. Man erhält eine stabile ölige Dispersion.

Die Applikation kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,025 bis 3 kg/ha, vorzugsweise 0,05 bis 0,5 kg/ha.

Die Wirkung der Cyclohexenonderivate der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,06 bis 0,125 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein

Aufgang der Pflanzen bzw. völlige Zerstörung zumindest der oberirdischen Teile.

Die in den Gewächshausversuchen verwendeten Pflanzen setzen sich aus folgenden Arten zusammen:

Alopecurus myosuroides (Ackerfuchsschwanz), Avena fatua (Flughafer), Avena sativa (Hafer), Digitaria sanguinalis (Blutfingerhirse), Echinochloa crus-galli (Hühnerhirse), Glycine max (Sojabohnen), Lolium multiflorum (Ital. Raygras), Setaria italica (Kolbenhirse), Sinapis alba (Weißer Senf), Sorghum halepense (Wilde Mohrenhirse), Sorghum bicolor (Mohrenhirse, Kulturhirse), Zea mays (Mais).

Als Vergleichsmittel wurden die aus der DE-A-3 032 973 bekannten Verbindungen 2-(1-Allyloxyamino-n-butyl)-5-(cyclohex-1-en-4-yl)-3-hydroxy-cyclohex-2-en-1-on (A) und 2-(1-Ethoxyimino-n-butyl)-5-(cyclohex-1-en-4-yl)-3-hydroxy-cyclohex-2-en-1-on (B) sowie die aus der DE-A-3 219 490 bekannte Verbindung 2-(1-Ethoxyamino-n-butyl)-5-(cyclooct-1-en-5-yl)-3-hydroxy-cyclohex-2-en-1-on (C) bzw. diese Verbindungen enthaltende herbizide Mittel geprüft.

Bei Vorauflaufanwendung erweisen sich beispielsweise die Verbindungen Nr. 4, 6, 11 und 13 als herbizid wirksam gegen Pflanzen aus der Familie der Gräser, während Sinapis alba als ein Vertreter dikotyler Pflanzen völlig ungeschädigt bleibt.

Weiterhin zeigen z. B. die Verbindungen Nr. 4 und 6 mit 0,125 kg Wirkstoff/ha bei Nachauflaufbehandlung starke herbizide Aktivität gegen ein breites Ungrasspektrum; die Sojabohne als dikotyle Kulturpflanze erleidet keinerlei Schaden.

Es lassen sich beispielsweise mit den Verbindungen 26, 28, 52, 53, 55 und 61 unerwünschte Grasarten gut bekämpfen, während die breitblättrige Pflanze Luzerne (als Beispiel für eine Kulturpflanze) völlig ungeschädigt bleibt.

Die beispielhaft ausgewählte Verbindung Nr. 8 eignet sich schon in geringen Aufwandmengen zur Bekämpfung unerwünschter Grasarten und zeichnet sich durch Verträglichkeit für Weizen aus.

Mit den beispielhaft ausgewählten Verbindungen 1 und 2 lassen sich bei voller Verträglichkeit für Sojabohnen Problemgräser deutlich besser bekämpfen als mit den bekannten Vergleichsmitteln A und B.

Verglichen mit dem bekannten Mittel C liegt das herbizide Wirkniveau beispielsweise von Verbindung Nr. 50 deutlich höher, ohne dabei die Verträglichkeit für Luzerne zu beeinflussen.

In Anbetracht des erfaßbaren Wirkungsspektrums zur Unkrautbekämpfung, der Verträglichkeit für Kulturpflanzen oder der unerwünschten Beeinflussung des Wachstums derselben sowie angesichts der Vielfalt der Applikationsmethoden können die erfindungsgemäßen Verbindungen in einer großen Zahl von Kulturpflanzen eingesetzt werden.

In Betracht kommen beispielsweise folgende Kulturpflanzen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum , Gossypium vitifolium) | Baumwolle |

| Botanischer Name | Deutscher Name |
|---|---|
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceium | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais (post directed) |

Zur Verbreiterung des Wirkungsspektrums und zur Erzielung synergistischer Effekte können die neuen substituierten Cyclohexenonderivate sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolin-carbonsäuren und andere in Betracht.

Außerdem kann es von Nutzen sein, die Cyclohexenonderivate der Formel I bzw. sie enthaltende herbizide Mittel allein oder in Kombination mit anderen Herbiziden auch noch mit weiteren Pflanzenschutzmitteln gemischt

gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. Cyclohexenonderivate der Formel I

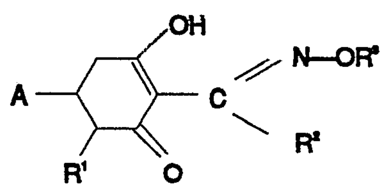

(I),

in der

A einen Cycloalkylrest mit 5 bis 12 Ringgliedern, an den ein oder zwei Oxiran- oder Thiiranringe kondensiert sind, und der gegebenenfalls durch bis zu vier Methylgruppen substituiert und/oder durch eine Alkylenkette mit maximal 3 C-Atomen überbrückt ist,
$R^1$ Wasserstoff, Methoxycarbonyl oder Cyano,
$R^2$ Alkyl mit 1 bis 4 C-Atomen und
$R^3$ Alkyl mit 1 bis 3 C-Atomen, Alkenyl mit 3 oder 4 C-Atomen, Halogenalkenyl mit 3 oder 4 C-Atomen und 1 bis 3 Halogensubstituenten oder Propargyl bedeuten,
und Salze dieser Verbindungen.

2. Cyclohexenonderivate der Formel I gemäß Anspruch 1 *dadurch gekennzeichnet*, daß $R^1$ Wasserstoff bedeutet.

3. Cyclohexenonderivate der Formel I gemäß Anspruch 1 *dadurch gekennzeichnet*, daß der Cycloalkylrest A 5 bis 8 Ringglieder aufweist.

4. Verfahren zur Herstellung eines Cyclohexenonderivates der Formel I gemäß Anspruch 1 *dadurch gekennzeichnet*, daß man eine Verbindung der Formel II

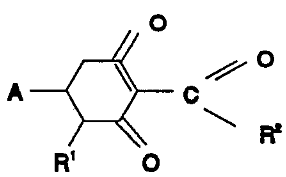

(II),

in der A, $R^1$ und $R^2$ die im Anspruch 1 genannten Bedeutungen haben,

a) mit einer Ammoniumverbindung der Formel $R^3O\text{-}NH_3Y$, in der $R^3$ die im Anspruch 1 genannten Bedeutungen hat und Y ein Anion bedeutet, in einem inerten Verdünnungsmittel gegebenenfalls in Gegenwart von Wasser bei einer Temperatur zwischen 0 und 80°C in Gegenwart einer Base oder
b) mit einem gegebenenfalls in wäßriger Lösung vorliegenden Hydroxylamin der Formel $R^3O\text{-}NH_2$, in der $R^3$ die im Anspruch 1 genannten Bedeutungen hat, in einem inerten Lösungsmittel umsetzt.

5. Herbizid, enthaltend ein Cyclohexenonderivat der Formel I gemäß Anspruch 1.

6. Herbizid, enthaltend inerte Zusatzstoffe und ein Cyclohexenonderivat der Formel I gemäß Anspruch 1.

7. Herbizid nach Anspruch 6, *dadurch gekennzeichnet*, daß es 0,1 bis 95 Gew.-% eines Cyclohexenonderivats der Formel I enthält.

8. Verfahren zur Bekämpfung unerwünschten Pflanzenwachstums, *dadurch gekennzeichnet*, daß man die unerwünschten Pflanzen oder von unerwünschtem Pflanzenwachstum freizuhaltende Flächen mit einer herbizid wirksamen Menge eines Cyclohexenonderivats der Formel I gemäß Anspruch 1 behandelt.

9. Tricarbonylverbindungen der Formel II

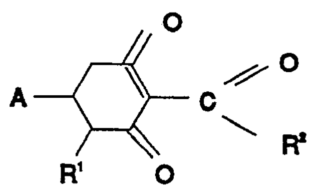

(II),

in der

A einen Cycloalkylrest mit 5 bis 12 Ringgliedern, an den ein oder zwei Oxiran- oder Thiiranringe kondensiert sind, und der gegebenenfalls durch bis zu vier Methylgruppen substituiert und/oder durch eine Alkylenkette mit maximal 3 C-Atomen überbrückt ist,
$R^1$ Wasserstoff, Methoxycarbonyl oder Cyano und
$R^2$ Alkyl mit 1 bis 4 C-Atomen bedeuten.

**Claims**

1. A cyclohexenone derivative of the formula I

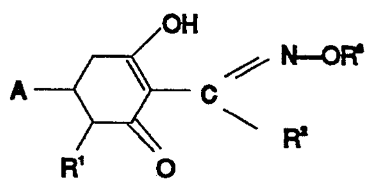

(I),

where
A is a cycloalkyl radical of 5 to 12 ring members which is fused with one or two oxirane or thiirane rings, is unsubstituted or substituted by not more than four methyl groups and/or is bridged with an alkylene chain of not more than 3 carbon atoms,
$R^1$ is hydrogen, methoxycarbonyl or cyano,
$R^2$ is alkyl of 1 to 4 carbon atoms and
$R^3$ is alkyl of 1 to 3 carbon atoms, alkenyl of 3 or 4 atoms, haloalkenyl of 3 or 4 carbon atoms which has 1, 2 or 3 halogen substituents or propargyl,
or a salt thereof.

2. A cyclohexenone derivative of the formula I as claimed in claim 1, wherein $R^1$ is hydrogen.

3. A cyclohexenone derivative of the formula I as claimed in claim 1, wherein cycloalkyl A has from 5 to 8 ring members.

4. A process for the preparation of a cyclohexenone derivative of the formula I as claimed in claim 1, wherein a

compound of the formula II

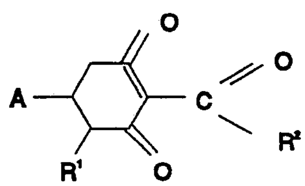

(II),

where A, $R^1$ and $R^2$ have the meanings given in claim 1, is reacted

a) with an ammonium compound of the formula $R^3O\text{-}NH_3Y$, where $R^3$ has the meanings given in claim 1 and Y is an anion, in an inert diluent, in the presence or absence of water at from 0 to 80°C in the presence of a base, or
b) in an inert solvent with a hydroxylamine of the formula $R^3O\text{-}NH_2$, where $R^3$ has the meanings given in claim 1, which may be present in aqueous solution.

5. A herbicide containing a cyclohexenone derivative of the formula I as claimed in claim 1.

6. A herbicide containing inert additives and a cyclohexenone derivative of the formula I as claimed in claim 1.

7. A herbicide as claimed in claim 6, containing from 0.1 to 95 % by weight of a cyclohexenone derivative of the formula I.

8. A method for controlling undesirable plant growth wherein the undesirable plants or the areas to be kept free from undesirable plant growth are treated with a herbicidally effective amount of a cyclohexenone derivative of the formula I as claimed in claim 1.

9. A tricarbonyl compound of the formula II

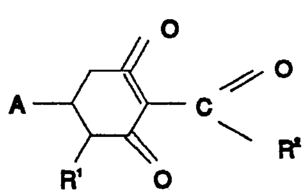

(II),

where
A is cycloalkyl of 5 to 12 carbon ring members which is fused with one or two oxirane or thiirane rings, is unsubstituted or substituted by not more than four methyl groups and/or is bridged with an alkylene chain of not more than 3 carbon atoms,
$R^1$ is hydrogen, methoxycarbonyl or cyano, and
$R^2$ is alkyl of 1 to 4 carbon atoms.

**Revendications**

1. Dérivés de cyclohexénone de formule I

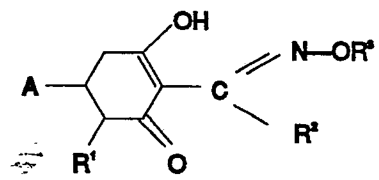

(I),

dans laquelle

A représente un reste cycloalkyle ayant 5 à 12 membres de cycle, sur lesquels sont condensés un ou deux noyaux oxirane ou thiirane, et qui est éventuellement substitué par jusqu'à quatre groupes méthyle et/ou ponté par une chaîne alkylène ayant au maximum 3 atomes C,
$R^1$ représente hydrogène, méthoxycarbonyle ou cyano
$R^2$ alkyle ayant 1 à 4 atomes C et
$R^3$ alkyle ayant 1 à 3 atomes C, alcényle ayant 3 à 4 atomes C, halogénoalcényle ayant 3 ou 4 atomes C et 1 à 3 substituants halogène ou propargyle
et sels de ces composés.

2. Dérivés de cyclohexénone de formule I selon la revendication 1, caractérisés par le fait que $R^1$ représente hydrogène.

3. Dérivés de cyclohexénone de formule I, caractérisés par le fait que le reste cycloalkyle A possède 5 à 8 membres de cycle.

4. Procédé de préparation d'un dérivé de cyclohexénone de formulel selon la revendication 1, caractérisé par le fait qu'on fait réagir un composé de formule II

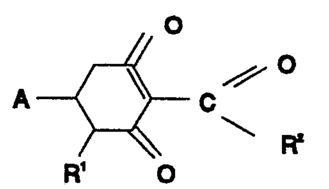

(II),

dans laquelle A, $R^1$ et $R^2$ ont les significations données dans la revendication 1

a) avec un composé d'ammonium de formule $R^3O\text{-}NH_3Y$, dans laquelle $R^3$ a la signification donnée dans la revendication 1 et Y représente un anion, dans un diluant inerte, éventuellement en présence d'eau, à une température comprise enter 0 et 80°C, en présence d'une base, ou
b) avec une hydroxylamine, se trouvant eventuellement en solution aqueuse, de formule $R^3O\text{-}NH_2$ dans laquelle $R^3$ a la signification indiquée dans la revendication 1, dans un solvant inerte.

5. Herbicide contenant un dérivé de cyclohexénone de formule I selon la revendication I.

6. Herbicide contenant des additifs inertes et un dérivé de cyclohexénone de formule I selon la revendication I.

7. Herbicide selon la revendication 6, caractérisé par le fait qu'il contient 0,1 à 95 % en poids d'un dérive de cyclohexénone de formule I selon la revendication I.

8. Procédé de lutte contre la croissance indésirable de plantes, caractérisé par le fait que l'on traite les plantes indésirables ou les surfaces à maintenir exemptes d'une croissance indésirable des plantes avec une quantité efficace, du point de vue herbicide, d'un dérivé de cyclohexénone de formule I selon la revendication I.

9. Composés tricarbonyles de formule II

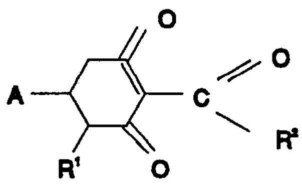

(II),

dans laquelle

A représente un reste cycloalkyle ayant 5 à 12 membres de cycle, sur lesquels sont condensés un ou deux noyaux oxirane ou thiirane, et qui est éventuellement substitué par jusqu'à quatre groupes méthyle et/ou ponté par une chaîne alkylène ayant au maximum 3 atomes C,

$R^1$ représente hydrogène, méthoxycarbonyle ou cyano et

$R^2$ alkyle ayant 1 à 4 atomes C.